# EUROPÄISCHE PATENTSCHRIFT

(11) **EP 1 845 957 B1**
(45) Veröffentlichungstag und Bekanntmachung des Hinweises auf die Patenterteilung: **22.12.2010**
(21) Anmeldenummer: 06723000.3
(22) Anmeldetag: 03.02.2006
(51) Int. Cl.: A61K 9/28, A61K 9/36, A61K 31/085, A61K 31/7034, A61K 36/15, A61K 36/481, A61K 36/482, A61K 36/708, A61P 19/10, A61P 25/22, A61P 25/24, A61P 29/00, A61P 35/00

(54) **VERFAHREN ZUR HERSTELLUNG EINES HYDROXYSTILBEN-HALTIGEN DROGENEXTRAKTES**
METHOD FOR PRODUCING A DRUG EXTRACT THAT CONTAINS HYDROXYSTILBENES
PROCEDE POUR PRODUIRE UN EXTRAIT MEDICAMENTEUX CONTENANT DES HYDROXYSTILBENES

(30) Priorität: 04.02.2005 DE 102005005268; 04.02.2005 DE 102005005271
(43) Veröffentlichungstag der Anmeldung: 24.10.2007
(73) Patentinhaber: Heger, Peter, 76698 Ubstadt-Weiher (DE); Rettenberger, Reinhard, 73033 Göppingen (DE); Spaich, Carl-Friedrich, 73092 Heiningen (DE)
(72) Erfinder: Heger, Peter, 76698 Ubstadt-Weiher (DE); Rettenberger, Reinhard, 73033 Göppingen (DE); Spaich, Carl-Friedrich, 73092 Heiningen (DE)
(74) Vertreter: Reitstötter - Kinzebach
(86) Internationale Anmeldenummer: PCT/EP2006/000955
(87) Internationale Veröffentlichungsnummer: WO 2006/082071

(56) Entgegenhaltungen:
- EP-A- 1 140 097
- EP-A- 1 161 944
- WO-A-97/44407
- WO-A-03/039557
- AU-A- 2 628 177
- FR-A- 2 835 185
- ANONYM: ""Phytoestrogen hilft bei Beschwerden im Klimakterium" ÄRZTE ZEITUNG, [Online] 16. Mai 2000 (2000-05-16), XP002378130 Gefunden im Internet: URL:http://www.aerztezeitung.de/docs/2000/ 05/16/089a1403.asp?cat=> [gefunden am 2006-04-24]
- ROTE LISTE SERVICE GMBH (ED.): "ROTE LISTE" 2002, EDITIO CANTOR VERLAG , AULENDORF , XP002379296 Eintrag 76001 "Phytoestrol N"
- AGGARWAL B B ET AL: "Role of resveratrol in prevention and therapy of cancer: Preclinical and clinical studies" ANTICANCER RESEARCH 2004 GREECE, Bd. 24, Nr. 5 A, 2004, Seiten 2783-2840, XP009065753 ISSN: 0250-7005 in der Anmeldung erwähnt
- SABICHI A L ET AL: "COX-2 inhibitors and other nonsteroidal anti-inflammatory drugs in genitourinary cancer" SEMINARS IN ONCOLOGY 2004 UNITED STATES, Bd. 31, Nr. SUPPL. 7, 2004, Seiten 36-44, XP009066086 ISSN: 0093-7754

## Beschreibung

Die Erfindung betrifft ein Verfahren zur Herstellung eines Hydroxystilben-haltigen Drogenextraktes aus einer pflanzlichen Droge und die Verwendung der auf diese Weise hergestellten Drogenextrakte zur Herstellung von verschiedenen Mitteln zu pharmazeutischen und nicht-pharmazeutischen Zwecken.

### HINTERGRUND DER ERFINDUNG

Unter dem Sammelbegriff Hydroxystilbene wird eine große Gruppe von teilweise pharmakologisch aktiven Verbindungen zusammengefasst, die als natürliche Inhaltsstoffe pflanzlicher Drogen oder durch chemische Synthese zugänglich sind.

So beschreiben beispielsweise Aggarwal et al in einem Übersichtsartikel (Anticancer Research, 24, 2004) Resveratrol und strukturell ähnliche Verbindungen sowie deren natürliche Quellen.

Aufgrund des potenziellen pharmakologischen Nutzens von natürlich vorkommenden Hydroxystilbenen und Hydroxystilbenmischungen besteht die Notwendigkeit, Verfahren zu entwickeln, welche eine zuverlässige und reproduzierbare Isolierung gewünschter Hydroxystilbene in technischem Maßstab und zudem in ausreichender Reinheit und Ausbeute ermöglichen.

Die FR 2 835 185 beschreibt einen komplexen Rhabarberextrakt, erhältlich aus Wurzelstöcken von Rheum rhaponticum, der dadurch gekennzeichnet sein soll, dass er mindestens 50 % Hydroxystilbene enthält, wobei diese Hydroxystilbene zu mindestens 50 % aus Rhaponticin, Desoxyrhaponticin, Astrangin und Piceatannol bestehen. Ein bevorzugter Extrakt enthält 15 - 50 Gew.-% Rhaponticin, 10 - 35 Gew.-% Desoxyrhaponticin, 5 -10 Gew.-% Astrangin und 0,1 - 3 Gew.-% Piceatannol. Dieser Extrakt wird, wie in den Beispielen veranschaulicht, durch wässrig-alkoholische Extraktion von Wurzelstöcken von Rheum rhaponticum hergestellt. Der damit erzielbare Gesamtanteil an Rhaponticin und Desoxyrhaponticin beträgt dabei nur 76 Gew.-%. Astrangin ist in einem Anteil von 11 Gew.-%, Piceatannol in einem Anteil von 3 Gew.-% und Anthracenoside sind in einem Anteil von 0,5 Gew.-% enthalten. Darüber hinaus scheint dieser Extrakt ca. 10 Gew.-% weitere, nicht näher beschriebene Bestandteile zu enthalten. In der FR 2 835 185 wird außerdem behauptet, dass der dortige spezielle Extrakt infolge angeblicher Synergieeffekte der verschiedenen Inhaltsstoffe des Extraktes biologische Eigenschaften besitzt, die der Wirkung der einzelnen Hydroxystilbene erheblich überlegen sind, insbesondere denjenigen Wirkungen, die die dort beschriebenen Inhaltsstoffe einzeln aufweisen sollen. Der dort beschriebene Extrakt soll angeblich antioxidative, tumorhemmende, entzündungshemmende und östrogene Eigenschaften besitzen. Tatsächlich liefert aber die FR 2 835 185 keine nachprüfbare technische Lehre für die behauptete pharmakologische Anwendbarkeit, geschweige denn, für den behaupteten Synergieeffekt des dort beschriebenen komplexen Drogenextraktes. Der experimentelle Teil beschreibt lediglich einzelne Formulierungsbeispiele für Kapseln, Tabletten oder Cremes. Insbesondere fehlen jegliche Versuchsdaten, welche die angebliche Brauchbarkeit zur Behandlung von solchen Erkrankungen belegen, welche im Zusammenhang mit freien Radikalen stehen, wie zum Beispiel beschleunigte Alterung, Krebs, Arteriosklerose, Falten, entzündliche Phänomene und dergleichen. Auch die behauptete Eignung einer Kombination des dort beschriebenen Rhabarberextrakts mit einem an Prenylflavonoiden reichen Hopfenextrakt zur Behandlung von mit freien Radikalen stehenden Krankheiten und/oder zur Behandlung von hormonellem Ungleichgewicht, wie Amenorrhoe, Menopause, Hitzewallungen usw. ist durch keine Daten belegt. Es bleibt darüber hinaus völlig unklar, welche der tatsächlich in dem dort beschriebenen Extrakt enthaltenen Komponenten (Rhaponticin, Desoxyrhaponticin, Astrangin, Piceatannol, Anthracenoside sowie die in einem Anteil von 10 % enthaltenen nicht analysierten Bestandteile) zu der behaupteten pharmakologischen Aktivität beitragen oder gegebenenfalls sogar für die behauptete Synergie zwingend erforderlich sind. Die tatsächliche Offenbarung der FR 2 835 185 sollte daher auf die Herstellung eines spezifischen, komplexen Rhabarberextraktes durch wässrig-alkoholische Extraktion von Wurzelstöcken von Rheum rhaponticum und die Herstellung spezifischer Hydroxystilben-Derivate sowie die Herstellung diverser pharmazeutischer Formulierungen beschränkt sein.

In zahlreichen weiteren Publikationen (vgl. z.B. Babu et al., Bioorg. Med. Chem Lett. 14 (2004), 3841 - 3845; Matsuda et al., Bioorg. Med. Chem. 9 (2001), 41-50) wird zur Extraktion der Inhaltsstoffe von Rheum Rhizoma die Verwendung von Methanol als Extraktionsmittel vorgeschlagen.

Die EP-A-1 140 097 beschreibt die Isolierung von Trihydroxystilben-Verbindungen aus Pflanzenmaterial durch Extraktion mit wässrigem Lösungsmittel, gefolgt von einer speziellen Kombination chromatographischer Schritte. Das wässrige Lösungsmittel ist insbesondere ein 75 % Alkohol umfassendes Alkohol-Wasser-Gemisch. Reines Wasser als Extraktionsmittel wird nicht verwendet. Typische extrahierte Pflanzen sind Vitis vinifera und Polygonum cuspidatum.

Die gemäß Stand der Technik erhaltenen Extrakte sind jedoch sehr komplex zusammengesetzt, sind als solches für eine medizinische Anwendung nur begrenzt geeignet und bedürfen weiterer Reinigungsschritte.

Es besteht daher Bedarf an einem verbesserten Verfahren zur Herstellung von Hydroxystilben-haltigen Drogenextrakten.

### ZUSAMMENFASSUNG DER ERFINDUNG

Aufgabe der vorliegenden Erfindung ist die Bereitstellung eines effizienten Verfahrens zur Herstellung eines Hydroxystilben-haltigen Drogenextraktes. Insbesondere ist Aufgabe der Erfindung einen Drogenextrakt herzustellen, welcher besonders rein ist und als überwiegende Hydroxystilben-Bestandteile die Glyconformen (Glycoside) von Hydroxystilbenen, wie insbesondere Rhaponticin und Desoxyrhaponticin, umfasst.

Obige Aufgabe konnte überraschenderweise durch Bereitstellung eines in den folgenden Abschnitten näher beschriebenen Extraktionsverfahrens gelöst werden.

### DETAILLIERTE BESCHREIBUNG DER ERFINDUNG

### 1. Bevorzugte Gegenstände der Erfindung

Gegenstand der Erfindung ist ein Verfahren zur Herstellung eines Hydroxystilben-haltigen Drogenextraktes, wobei man
a) einen Hydroxystilben-haltigen Teil einer Drogenpflanze, gegebenenfalls in nach üblicher Weise zerkleinerter Form, bereitstellt,
b) diesen mit einem wässrigen, organischen oder wässrig-organischen Extraktionsmittel versetzt,
c) nach Einwirken des Extraktionsmittels eine Flüssigextraktphase aus der Mischung gewinnt und die Extraktion gegebenenfalls mehrfach wiederholt, und
d) das Extraktionsmittel aus den so erhaltenen Flüssigextraktphasen entfernt, wobei man eine Extraktion mit einem wässrigen Extraktionsmittel bei einem PH-Wert der Mischung in alkalischen bereich durchführt.

Insbesondere ist Gegenstand ein Verfahren, bei dem der Hydroxystilben-haltige Teil einer Drogenpflanze wenigstens eine Piceatannol- und/oder Resveratrol-Prodrug enthält.

Hydroxystilbene in Sinne der Erfindung umfassen insbesondere zuckerhaltige (Glycoside) und zuckerfreie (Aglycone) Prodrugs (d.h. Vorläufer oder Derivate) von Resveratrol und Piceatannol. Resveratrol- und Piceatannol-Prodrugs im Sinne der Erfindung sind insbesondere auch Substanzen, welche in vivo, wie z.B. im Menschen und/oder einem anderen Säuger, wie z.B. Hund, in Resveratrol und/oder Piceatannol überführbar sind. Typische Glycoside sind dabei Rhaponticin, Desoxyrhaponticin und Astringin; typische Aglyconvorläufer sind Rhapontigenin und Desoxyrhapontigenin, ohne jedoch darauf beschränkt zu sein (vgl. auch Tabelle 1 und folgende Strukturformel).

Die Begriffe "Prodrug" oder "Vorläufer" sind aber im Kontext der Erfindung nicht als funktionelle Einschränkung zu verstehen. Wie durch verschiedene Versuchsergebnisse belegbar, entfalten insbesondere die erfindungsgemäßen "Vorläufer" per se ebenfalls vorteilhafte pharmakologische Wirkungen.

Erfindungsgemäß können Hydroxystilbene als Salz oder in phenolischer Form, in einer stereoisomeren Form, wie cis- oder trans-Form, oder als Gemisch solcher stereoisomeren Formen in der Droge enthalten sein, bzw. als Verfahrensprodukt erhalten werden.

**Tabelle 1**

| **Hydroxystilben** | **Chemischer Name** | **CAS Nr.** |
|---|---|---|
| Rhaponticin | 3,3',5-Trihydroxy-4'-methoxystilben-3-O-ß-D-glucopyranosid | 155-58-8 |
| Desoxyrhaponticin | 3',5-Dihydroxy-4'-methoxystilben-3-O-β-D-glucopyranosid | 30197-14-9 |
| Rhapontigenin (Trans-Rhapontigenin) | 3,3',5-Trihydroxy-4'-methoxystilben | 500-65-2 |
| Desoxyrhapontigenin | 3',5-Dihydroxy-4'-methoxystilben | 33626-08-3 |

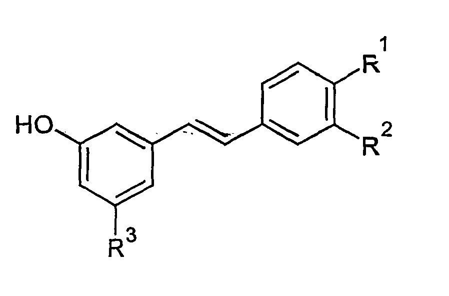

| | R¹ | R² | R³ |
|---|---|---|---|
| Resveratrol | OH | H | OH |
| Rhaponticin | OCH₃ | OH | O-Glc |
| Desoxyrhaponticin | OCH₃ | H | O-Glc |
| Rhapontigenin | OCH₃ | OH | OH |
| Desoxyrhapontigenin | OCH₃ | H | OH |
| Astringin | OH | OH | O-Glc |
| Piceatannol (Astringenin) | OH | OH | OH |

Insbesondere betrifft die Erfindung ein Verfahren, wobei der erhaltene Extrakt wenigstens eine Verbindung, ausgewählt unter Rhaponticin, Desoxyrhaponticin, Rhapontigenin, Desoxyrhapontigenin als Salz oder in phenolischer Form, in einer stereoisomeren Form davon, wie cis- oder trans-Form, oder als Gemisch solcher stereoisomeren Formen umfasst.

Vorzugsweise liegen die extrahierten Hydroxystilbene aber im wesentlichen in der trans-Form vor. Salze sind insbesondere die Alkali- und Erdalkaliphenolate obiger Verbindungen, die eine oder mehrere freie phenolische Hydroxylgruppen aufweisen. Liegen mehrere Hydroxylgruppen vor, so können diese teilweise oder vollständig in der Salzform vorliegen.

Die anfallenden Pflanzenextrakte oder einzelne Komponenten davon können auch Derivatisierungsreaktionen unterzogen werden, um sogenannte funktionelle Derivate zu erhalten. Dabei handelt es sich insbesondere um solche Derivate, die im menschlichen oder tierischen Körper nach Verabreichung wieder in die nichtderivatisierte Ausgangsverbindung zurückführbar sind. Insbesondere sind zu nennen Ether und Esterderivate der erfindungsgemäß verwendeten Verbindungen. Dabei können einzelne oder alle veretherbaren oder veresterbaren Gruppen eines Moleküls (insbesondere die phenolischen und glykosidischen Hydroxylgruppen) derivatisiert sein. Beispiele geeigneter Derivate und deren Herstellung sind z.B. in der FR 2 835 185 beschrieben, worauf hiermit ausdrücklich Bezug genommen wird. So können genannt werden, ohne darauf beschränkt zu sein: Ester von gesättigten oder ungesättigten, aliphatischen oder aromatischen Carbonsäuren mit bis zu 25 Kohlenstoffatomen, wie 1 bis 25 Kohlenstoffatomen, wie z.B. gesättigte C₆ - C₂₂-Fettsäuren (wie z.B. gesättigte unverzweigte Fettsäuren ausgewählt unter Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure); oder Silylether, wobei das Siliziumatom drei gleiche oder verschiedene, geradkettige oder verzweigte, gesättigte oder ungesättigte Kohlenwasserstoffreste mit bis zu 20 Kohlenstoffatomen, wie z.B. C₁ - C₂₀-Alkyl oder C₂ - C₂₀-Alkenyl, trägt.

Bevorzugt wird eine Wirkstoffkombination aus wenigsten zweien der oben genannten Verbindungen erhalten, wie z.B. 2, 3, 4, 5, 6, 7 oder 8 Einzelverbindungen, wobei die Gruppe der Resveratrol-Vorläufer (insbesondere Desoxyrhaponticin und Desoxyrhapontigenin) und der Piceatannol-Vorläufer (insbesondere Rhaponticin und Rhapontigenin) durch jeweils eine Verbindung vertreten ist.

In einer weiteren bevorzugten Ausführungsform des erfindungsgemäßen Verfahrens wird ein Extrakt bereitgestellt, der einen hohen Anteil an Glycosiden, insbesondere Glycosiden des oben beschriebenen Typs, aufweist, wie z.B. einen Anteil von 30 bis 100 Gew.-%, 50 bis 100 Gew.-%, 60 bis 99 Gew.-% oder 80 bis 98 Gew.-% oder 85 bis 96 Gew.-%, jeweils bezogen auf das Gesamtgewicht des erhaltenen Trockenextrakts.

Ein "Trockenextrakt" in Sinne der Erfindung liegt insbesondere dann vor, wenn der Restfeuchtegehalt, d.h. der Restanteil an Wasser und/oder organischer Flüssigkeit, (wie Extraktionsmittel) weniger als etwa 5 Gew.-%, insbesondere weniger als 2 Gew.-%, wie z.B. 0 bis 1,5 Gew.-% oder 0,1 bis 0,5 Gew.-%, jeweils bezogen auf das Gesamtgewicht des erhaltenen Trockenextrakts, beträgt.

In einer weiteren bevorzugten Ausführungsform wird ein Extrakt bereitgestellt der im wesentlichen frei ist von Aglycon-Derivaten von Rhaponticin und Desoxyrhaponticin, wie insbesondere Resveratrol und Piceatannol. "Im Wesentlichen frei" bedeutet einen Aglycon-Gehalt von weniger als 5 Gew.-%, insbesondere weniger als 2 Gew.-% wie z.B. weniger als 1 Gew.-% oder 0,1 Gew.-%, wie 0 bis 0,05 Gew.-%, jeweils bezogen auf den Gesamtgehalt an Rhaponticin und Desoxyrhaponticin.

Weiterhin werden Wirkstoffkombinationen bevorzugt hergestellt, die einen Hydroxystilben-Gesamtgehalt, insbesondere einen Gesamtgehalt an Desoxyrhaponticin, Desoxyrhapontigenin, Rhaponticin und Rhapontigenin, oder einen Gesamtgehalt an Rhaponticin und Desoxyrhaponticin, von mehr als 90 Gew.-%, wie z.B. 91 bis 100 Gew.-%, oder 92 bis 99 oder 93 bis 98 oder 94 bis 97 Gew.-%, aufweisen.

Weiterhin werden solche Wirkstoffkombinationen bevorzugt hergestellt, die einen Gehalt von weniger als 0,5 Gew.-% , wie z.B. 0 - 0,49 Gew.% oder 0,001 bis 0,3 oder 0,01 bis 0,2 oder 0,01 bis 0,1 Gew.-% an Anthrachinon und/oder Anthrachinoiden (jeweils bezogen auf Trockengewicht der Wirkstoffkombination) aufweisen. Anthrachinoide sind dabei im weitesten Sinn als Substanzen mit Anthrachinon-Grundgerüst zu verstehen.

In einer bevorzugten Ausführungsform ist die Drogenpflanze ausgewählt unter natürlichen Pflanzen sowie durch Züchtung veränderten oder rekombinanten, genetisch veränderten, Pflanzen, die einen in Vergleich zur entsprechenden unveränderten Pflanze höheren Gehalt an wenigstens einem der gewünschten Inhaltsstoffe aufweisen. Insbesondere sind diese Pflanzen ausgewählt unter Pflanze der Gattung Rheum spp., Astragalus spp., Cassia spp. oder Picea spp. oder wirkstoffhaltigen Pflanzenteilen. Nichtlimitierende Beispiele für geeignete Arten dieser Gattungen sind Rheum undulatum, Rheum palmatum, Rheum tataricum, Rheum officinale, Rheum wittrockii, Rheum altaicum, Rheum reticulatum, Astragalus complanatus Cassia garrettiana und Picea sitchensis. Bevorzugt ist außerdem der Einsatz sortenreiner Drogenpflanzen.

Dem Fachmann ist außerdem bewusst, dass Gattungen/Arten unterschiedlicher Provinienz sowie verschiedenen Alters (d.h. Ernte zu verschiedenen Zeiten der Vegetationsperiode) einsetzbar sind, was wiederum Einfluss auf Art, Menge und Zusammensetzung des daraus isolierbaren Hydroxystilbene und Gemische haben kann. Ebenso sind verschiedene Pflanzenteile, wie Wurzeln, Rhizome, Blätter und/oder Stängel, grundsätzlich brauchbar.

Das jeweilige Pflanzenteil oder Gemisch von Pflanzenteilen kann, falls zweckmäßig vor der Extraktion mechanisch behandelt, wie z.B. gemahlen, gehackt, gehaspelt, gestampft oder geschnitten, werden. Falls zweckmäßig kann auch eine Vortrocknung, wie z.B. 2 Stunden bis 2 Tage bei 30 bis 50°C, erfolgen um den Flüssigkeitsgehalt zu reduzieren.

Insbesondere ist der zur Extraktion verwendete, Hydroxystilben-haltige Teil der Drogenpflanze die Wurzel der Drogenpflanze, wie z.B. von Rheum rhaponticum.

Gegenstand der Erfindung ist insbesondere ein Verfahren bei dem man von der Droge ein Hydroxystilben-haltiges Perkolat herstellt. Unter einer "Perkolation" versteht man ein kontinuierliches Ausziehen löslicher Stoffe einer Droge durch laufende Erneuerung des Lösungsmittels. Dadurch besteht dauernd ein Konzentrationsgefälle, so dass ein großer Teil aller löslichen Stoffe in den Auszug übergeht.

Alternativ ist auch eine kontinuierliche oder periodische Durchmischung des Ansatzes, wie z.B. durch Rühren oder Schütteln, möglich.

Die Temperatur während der erfindungsgemäßen Extraktion liegt gewöhnlich im Bereich von 10 bis 50°C, wie z.B. 25 bis 35°C. Der Druck liegt gewöhnlich bei Normaldruck. Falls eine Beschleunigung der Extraktionsgeschwindigkeit oder Extraktqualität erzielbar ist, kann der Druck während der Extraktion auch variiert, wie z.B. erhöht oder verringert, werden.

Die Extraktionsdauer kann dabei je nach den gewählten Bedingungen, wie Art der Droge, Ansatzgröße, verwendetem Extraktionsmittel und Temperatur, 1 Stunde bis mehrere Tage, wie z.B. 10 bis 72 Stunden erfordern.

Der Extraktionsvorgang kann erforderlichenfalls mehrfach wiederholt werden, um eine möglichst vollständige Isolierung insbesondere der gewünschten Inhaltsstoffe zu gewährleisten. Das Gewichtsverhältnis von vorgelegter Droge zu flüssigem Extraktionsmittel kann über einen weiten Bereich sowie von Extraktionsschritt zu Extraktionsschritt variieren. Typischerweise liegt das Gewichtsverhältnis von Droge zu Extraktionsmittel im Bereich von 10:1 1 bis etwa 1:200 oder etwa 1:2 bis 1:50, oder 1:4 bis 1:10.

Die Extraktion führt man mit einem wässrigen, im wesentlichen an organischem Lösungsmittel freiem Extraktionsmittel, wie insbesondere Wasser, vorzugsweise gereinigtem Wasser, bei einem pH-Wert der Mischung im alkalischen Bereich durch, wobei der pH-Wert der Mischung insbesondere im Bereich von etwa 11 bis 12, wie z.B. bei etwa 11,3 bis 11,8 liegt.

Dabei wird der pH-Wert der Mischung beispielsweise mit Hilfe einer anorganischen Base, ausgewählt unter Alkali- und Erdalkalimetallhydroxiden, wie z.B. Calciumhydroxid oder Calciumoxid, eingestellt. Zu diesem Zweck kann man beispielsweise eine konzentrierte Löschkalklösung herstellen, indem man 3 bis 8 Teile CaO in 20 Teilen gereinigtem Wasser löst. Diese Lösung ist stark alkalisch und weist einem pH-Wert im Bereich von etwa 12 bis 13, wie z.B. von etwa 12,4 bis 12,6, auf.

Das Mengenverhältnis von vorgelegter Droge zu Base, wie z.B. Calciumhydroxid (berechnet als Calciumoxid) kann erfindungsgemäß im Bereich von etwa 5:1 bis 20:1, wie etwa 8:1 bis 12:1 oder 9:1 bis 11:1, liegen.

Bevorzugt wird das Verfahren so durchgeführt, dass man aus der erhaltenenen alkalischen Flüssigextraktphase die gewünschten Hydroxystilbene ausfällt, beispielsweise indem man den pH-Wert des Extrakts auf einen Wert im Bereich von etwa 3 bis 4, wie z.B. 3,2 bis 3,8 oder 3,4-3,6, einstellt und gegebenenfalls anschließend den Niederschlag abtrennt, gegebenenfalls wäscht und gegebenenfalls trocknet.

Zur Ansäuerung verwendet man dabei eine beliebige anorganische oder organische Säure, wie z.B. Salzsäure oder Schwefelsäure, insbesondere aber organische Säuren wie Ameisensäure oder Essigsäure.

Vor dem Abtrennen des Niederschlags kann es zweckmäßig sein, den Ansatz einige Stunden oder Tage ruhen zu lassen, um eine möglichst quantitative Fällung der gewünschten extrahierten Inhaltsstoffe zu erreichen.

Das Waschen des Niederschlags kann z.B. mit gereinigtem Wasser erfolgen und dient insbesondere zur Entfernung restlicher Säure.

Durch Trocknen, z.B. bei 30 bis 50°C oder 35 bis 45°C, beispielsweise über einen Zeitraum von 1 bis 100 Stunden, wird restliche Flüssigkeit aus dem Extrakt entfernt, bis die Restfeuchte im oben angegebenen Bereich liegt. Die Trocknung erfolgt in an sich bekannter Weise, z.B. in einem Trockenschrank. Eine Gefriertrocknung ist ebenfalls möglich.

Der erfindungsgemäß hergestellte Extrakt kann ohne weitere Nachbehandlung direkt zur Herstellung des jeweils gewünschten Mittels eingesetzt werden. Eine Weiterbehandlung der Inhaltsstoffe, wie z.B. eine Derivatisierung, wie z.B. Veresterung, Deglycosylierung, Überführung in ein geeignetes Salz, ist aber grundsätzlich möglich.

Ein weiterer Gegenstand der Erfindung ist die Verwendung eines Hydroxystilben-haltigen Drogenextraktes, erhältlich nach einem Verfahren gemäß obiger Definition zur Herstellung eines Mittels, wie z. B. ausgewählt unter Arzneimitteln, wie Homöopathika, anderen Arzneipflanzenzubereitungen, Nahrungsergänzungsmitteln und diätetischen Lebensmitteln.

Insbesondere kann das Arzneimittel oder pharmazeutische Mittel eine feste Dosierungsform umfassen.

Diese feste Dosierungsform umfasst insbesondere einen wirkstoffhaltigen festen Kern mit einem pharmazeutisch verträglichen Träger und einem Wirkstoffgehalt von etwa 1 bis 20 Gew.-%, wie z.B. 2 bis 15 oder 5 bis 10 Gew.-%, bezogen auf das Gesamtgewicht des Kerns, wobei der "Wirkstoff" einen in obiger Weise hergestellten Hydroxystilben-haltigen Extrakt umfasst, der Hydroxystilben-Inhaltsstoffe der Droge gemäß obiger Definition, insbesondere Piceatannol- und/oder Resveratrol-Prodrugs enthält.

Die Wirkstoffkombination im erfindungsgemäßen Mittel umfasst insbesondere im wesentlichen Rhaponticin und Desoxyrhaponticin in einem Gewichtsverhältnis von etwa 2:1 bis 1:2.

Beispiele für erfindungsgemäß erhältliche Wirkstoffkombinationen bestehen im wesentlichen aus etwa
60-66 Gew.-% Rhaponticin
30-35 Gew.-% Desoxyrhaponticin
0-2 Gew.-% Rhapontigenin und
0-2 Gew.-% Desoxyrhapontigenin.

Erfindungsgemäß kann eine Dosierungsform einem Gesamt-Wirkstoffgehalt von etwa 2 bis 20 mg, wie z.B. 3 bis 15 oder 4 bis 10 mg, je Dosiseinheit aufweisen.

Eine bevorzugte feste Dosierungsform weist weiterhin einen Lactose-freien Kern auf.

Bevorzugt hergestellte feste Dosierungsformen liegen in Form einer Pille, einer Tablette, eines Extrudats oder eines Granulats vor.

Eine weitere bevorzugte feste Dosierungsform hat außerdem einen magensaftresistenten Überzug.

Ein weiterer Gegenstand der Erfindung ist die Verwendung eines Hydroxystilben-haltigen Drogenextrakts, hergestellt nach einem Verfahren gemäß obiger Definition, zur Herstellung eines Mittels zur Behandlung von klimakterischen Beschwerden der Frau, juveniler Oligomenorrhoe und Dysmenorrhoe, primärer und sekundärer Amenorrhoe oder Endometritis, von Prostatakrebs und Erkrankungen des unteren Urogenitaltrakts, von Krebs, von chronischen entzündlichen Erkrankungen, yon Depressionen und Angst, von Osteoporose sowie von Kopfschmerzen und Migräne, insbesondere zur Behandlung von klimakterischen Beschwerden in der Peri- oder Postmenopause, wie insbesondere von Hitzewallungen, Schweißausbrüchen, Schlafstörungen, Reizbarkeit, physischer und mentaler Erschöpfung, Sexualproblemen und Harntraktbeschwerden.Die erfindungsgemäß hergestellten Mittel eignen sich insbesondere zur Behandlung von klimakterischen Beschwerden aufgrund natürlicher oder therapeutisch verursachter Menopause.

Aufgrund der ausgezeichneten Verträglichkeit der oben beschriebenen Wirkstoffe bzw. Wirkstoffkombinationen ist Gegenstand der Erfindung auch die Verwendung im Rahmen einer Langzeittherapie, die zeitlich unbegrenzt erfolgen kann. Die dabei zu verabreichende tägliche Dosis kann im Bereich von 0,1 bis 20 mg oder 0,5 bis 15 mg, 1 bis 10 oder 4 bis 8 mg Wirkstoff oder Wirkstoffkombination, wie z.B. ERr 731^{®} liegen.

Gegenstand der Erfindung ist insbesondere auch ein Verfahren zur Herstellung der oben bezeichneten Mittel, wobei man zunächst ein Extraktionsverfahren gemäß obiger Definition durchführt und den dabei anfallenden Hydroxystilben-haltigen Drogenextrakt in an sich bekannter Weise, zusammen mit üblichen Hilfsstoffen zu einem der oben genannten Mittel formuliert.

### 2. Weitere spezielle Ausgestaltungen erfindungsgemäßhergestellter Formulierungen

### 2.1 Arzneimittel

Die Erfindung umfasst auch die Herstellung pharmazeutischer Mittel (Arzneimittel), wie Homöophatika, zur Behandlung eines Individuums, vorzugsweise eines Säugers, insbesondere eines Menschen, Nutz- oder Haustieres. So werden die oben beschriebenen Wirkstoffe oder Wirkstoffkombinationen gewöhnlich in Form von pharmazeutischen Zusammensetzungen verabreicht, die einen pharmazeutisch verträglichen Exzipienten mit wenigstens einem erfindungsgemäßen Wirkstoff, insbesondere einem Gemisch mehrerer erfindungsgemäßer Wirkstoffe, und gegebenenfalls weitere Wirkstoffe umfassen. Diese Zusammensetzungen können beispielsweise auf oralem, lokalem, rektalem, transdermalem, subkutanem, intravenösem, intramuskulärem, intraperitonealem, intrakutanem oder intranasalem Weg verabreicht werden.

Beispiele geeigneter pharmazeutischer Formulierungen sind feste Arzneiformen, wie Pulver, Puder, Granulate, Tabletten, wie überzogene Tabletten, magensaftresistente überzogene Tabletten, Mantel-, Punkt- und Schichttabletten, Pastillen, Kautabletten, Lutschtabletten, Sachets, Cachets, Dragees, Kapseln wie Hart- und Weichgelatinekapseln, Globuli, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen, Nasentropfen, Nasenspray und Tinkturen. Auch implantierte Abgabevorrichtungen können zur Verabreichung erfindungsgemäßer Inhibitoren verwendet werden. Ferner können auch Liposomen, Mikrosphären oder Polymermatrices zur Anwendung kommen.

Bei der Herstellung der Zusammensetzungen werden erfindungsgemäße Wirkstoffe oder Wirkstoffkombinationen gewöhnlich mit einem Exzipienten vermischt oder verdünnt. Exzipienten können feste, halbfeste oder flüssige Materialien sein, die als Vehikel, Träger, Adsorbens oder Medium für den Wirkstoff oder die Wirkstoffkombinationen dienen.

Zu geeigneten Exzipienten gehören beispielsweise Lactose, Dextrose, Succrose, Sorbitol, Mannitol, Stärken, Akaziengummi, Calciumphosphat, Alginate, Traganth, Gelantine, Calciumsilikat, mikrokristalline Cellulose, Polyvinylpyrrolidon, Cellulose, Cellulosederivate, wie z.B. Methylcellulose, Wasser, Sirupe und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen.

Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist; vgl auch Hager's Handbuch der Pharmazeutischen Praxis, Springer Verlag, Heidelberg.

In einer bevorzugten Ausführungsform wird ein pharmazeutisches Mittel bereitgestellt, das eine feste Dosierungsform umfasst. Diese feste Dosierungsform umfasst ihrerseits einen wirkstoffhaltigen festen Kern mit einem pharmazeutisch verträglichen Träger und einem Wirkstoffgehalt von etwa 1 bis 20 Gew.-%, bezogen auf das Gesamtgewicht des Kerns, wobei der Wirkstoff oder die Wirkstoffkombination eine Verbindung, ausgewählt unter Resveratrol- und Piceatannol-Prodrugs; sowie Resveratrol und Piceatannol; sowie deren stereoisomeren Formen, jeweils in Form ihrer Salze oder in der Phenolform, oder Kombinationen von wenigstens zwei dieser Verbindungen, umfasst. Bevorzugte Wirkstoffkombinationen sind wie oben definiert.

Diese feste Dosierungsform hat beispielsweise einem Gesamt-Wirkstoffgehalt von etwa 1 bis 20 mg, wie z. B. 2 bis 10 mg, je Dosiseinheit und kann in Form einer Pille, einer Tablette, eines Extrudats oder eines Granulat vorliegen und z.B. dragiert sein. Gewünschtenfalls kann sie auch einen magensaftresistenten Überzug aufweisen.

Die feste Dosierungsform wird z.B. hergestellt, indem man den Wirkstoff oder die Wirkstoffkombination mit dem pharmazeutisch verträglichen Träger mischt und die Mischung zum Wirkstoffkern verfestigt. Dabei löst oder dispergiert man den Wirkstoff oder die Wirkstoffkombination in einer inerten Flüssigkeit, mischt ihn/sie mit dem Träger und entfernt das Lösungsmittel während oder nach der Verfestigung. Den Wirkstoffkern kann man dann gegebenenfalls mit einem magensaftresistenten Überzug versehen, bevor man den Kern in üblicher Weise dragiert. Beispielsweise sind derartige Überzüge frei von Weichmachern, wie Phthalaten, wie z.B. Diethylphthalat. Beschichtungsmittel, welche sich besonders zur Herstellung magensaftresistenter, weichmacherfreier Überzüge eignen, sind ausgewählt unter bekannten natürlichen und synthetischen Beschichtungsmitteln (vgl. z.B. Voigt, Pharmazeutische Technologie, 7. Auflage 1993, Ullstein Mosby, Berlin). Besonders geeignete Beschichtungsmittel sind, ohne darauf beschränkt zu sein, Schellack und Cellulosederivate, wie Hydroxypropylmethylcellulosederivate, wie z.B. Hydroxypropylmethylcelluloseacetatsuccinat, erhältlich unter der Handelsbezeichnung AQOAT.

Insbesondere ist eine feste Dosierungsform mit einem Gesamtgewicht im Bereich von etwa 150 mg ± 20 mg einem Kerngewicht von 84 mg ± 10 mg und einem Wirkstoffgehalt von etwa 3 bis 10 mg zu nennen.

Geeignet sind weiterhin feste Dosierungsformen, wobei diese eine Gleichförmigkeit des Wirkstoffgehalts (gemittelt über 10 oder 20 zufällig ausgewählte einzelne Dosiseinheiten) maximal ± 5 Gew.-%, wie z.B. ± 0,1 bis 4 oder ± 0,5 bis 3 oder ± 1 bis 2 Gew.-%, bezogen auf den Wirkstoffgehalt in der Dosierungsform aufweisen (z.B. bestimmt nach Ph. Eur. 5. Ausgabe 2005 (5.0/2.09.06.00)).

Erfindungsgemäße flüssige Dosierungsformen werden z.B. dadurch hergestellt, indem man den oder die Wirkstoffe, wie z.B. einen ERr731^{®} Trockenextrakt, in einem geeigneten Lösungsmittel, wie. z.B einem Wasser/Alkoholgemisch, ggf. zusammen mit weiteren üblichen Zusätzen, löst. Wirkstoffgehalte von 0,1 bis 20 oder 1 bis 10 mg/ml werden dabei gewöhnlich eingestellt.

Erfindungsgemäße halbfeste Dosierungsformen, wie z.B. Gele stellt man beispielsweise her, indem man den oder die Wirkstoffe, wie z.B. einen ERr 731^{®} Trockenextrakt, in einem geeigneten Lösungsmittel, wie z.B einem Wasser/Alkoholgemisch, Alkohol oder Glycerin, löst und die Lösung in den bereits gequollenen Gelbildner, ggf. zusammen mit weiteren üblichen Zusätzen einarbeitet. Wirkstoffgehalte von 1 bis 12 oder 2 bis 6 mg pro Gramm der Formulierung werden dabei gewöhnlich eingestellt.

Erfindungsgemäß geeignete Lösungsmittel für die Herstellung von Formulierungen sind insbesondere zu nennen ein- oder mehrwertige Alkohole, wie insbesondere Ethanol, Glycerin und Mischungen davon mit Wasser, wie z.B. 10 bis 50 Vol.-% Ethanol in Wasser.

Die Herstellung erfindungsgemäßer Darreichungsformen bzw. pharmazeutischer Mittel erfolgt unter Anwendung allgemein bekannter Methoden der pharmazeutischen Technologie, wie z.B. beschrieben in Voigt, Pharmazeutische Technologie, 7. Auflage 1993, Ullstein Mosby, Berlin.

Art und Dauer der Verabreichung der erfindungsgemäßen Arzneimittel obliegen der Entscheidung des behandelnden Arztes. Dieser kann in Abhängigkeit vom gewählten Verabreichungsweg, von der Wirksamkeit der konkreten Wirkstoffzusammensetzung, der Art und Schwere der zu behandelnden Erkrankung, vom Befinden des Patienten und von dessen Ansprechen auf die Therapie eine geeignete Dosis und einen entsprechenden Dosierungsplan festlegen. Beispielsweise kann eine geeignete Einzeldosis etwa 0,1 bis 50 mg, wie z.B. 2 bis 12 oder 2 bis 20 mg, Wirkstoff oder Wirkstoffkombination gemäß obiger Definition enthalten und 1 bis 3-mal täglich verabreicht werden, bis der gewünschte Behandlungserfolg zu beobachten ist.

### 2.2 Nahrungsergänzungsmittel und Lebensmittel

Zu den erfindungsgemäßen Mitteln gehören insbesondere auch Nahrungsergänzungsmittel und Lebensmittel, insbesondere funktionale oder diätetische Lebensmittel. Die erfindungsgemäßen Lebensmittel besitzen neben einer vorwiegend nährwertbezogenen Funktion zusätzlich eine wirkstoffbezogene Funktion, welche insbesondere die erfindungsgemäße Wirkstoffkombination betrifft. Sie werden daher als funktionale oder diätetische Lebens- oder Nahrungsmittel bezeichnet. Nahrungsergänzungsmittel dienen zur Ergänzung der täglichen Ernährung mit der erfindungsgemäßen Wirkstoffkombination, wobei die nährwertbezogene Funktion des Nahrungsergänzungsmittels für sich genommen in den Hintergrund tritt.

Die Formulierungsgrundlage für erfindungsgemäße Nahrungsergänzungsmittel und Lebensmittel umfasst ebenfalls physiologisch akzeptable Hilfsstoffe im weitesten Sinn, wie z.B. oben genannte Exzipienten. Hilfsstoffe im erfindungsgemäßen Sinne können auch einen Nährwert besitzen und deshalb allgemein als Nahrungskomponente verwendet werden. Auch Nährstoffe, insbesondere essentielle Nährstoffe, können dazu gehören.

Nahrungskomponenten enthalten in der Regel eine oder mehrere Aminosäuren, Kohlenhydrate oder Fette und sind für die menschliche und/oder tierische Ernährung geeignet. Sie umfassen als Einzelkomponenten, häufig pflanzliche aber auch tierische Produkte, insbesondere Zucker gegebenenfalls in Form von Sirupen, Fruchtzubereitungen, wie Fruchtsäfte, Nektar, Fruchtpulpen, Pürees oder getrocknete Früchte, beispielsweise Apfelsaft, Grapefruitsaft, Orangensaft, Apfelmus, Tomatensauce, Tomatensaft, Tomatenpüree; Getreideprodukte, wie Weizenmehl, Roggenmehl, Hafermehl, Maismehl, Gerstenmehl, Dinkelmehl, Maissirup, sowie Stärken der genannten Getreide; Milchprodukte, wie Milcheiweiß, Molke, Joghurt, Lecithin und Milchzucker.

Beispiele essentieller Nährstoffe sind insbesondere Vitamine, Provitamine, Mineralstoffe, Spurenelemente, Aminosäuren und Fettsäuren. Als essentielle Aminosäuren seien genannt Isoleucin, Leucin, Lysin, Methionin, Phenylalanin, Threonin, Tryptophan und Valin. Dazu gehören auch semi-essentielle Aminosäuren, die beispielsweise in Wachstumsphasen oder Mangelzuständen zugeführt werden müssen, wie Arginin, Histidin, Cystein und Tyrosin. Als Spurenelemente seien genannt: essentielle Spurenelemente und Mineralstoffe, wie: Eisen, Kupfer, Zink, Chrom, Selen, Calcium, Magnesium, Natrium, Kalium, Mangan, Cobalt, Molybdän, Iod, Silicium, Fluor, Chlor, Phosphor, Zinn, Nickel, Vanadium, Arsen, Lithium, Blei, Bor. Als für den Menschen essentielle Fettsäuren seien genannt: Linolsäure und Linolensäure, ARA (Arachidonsäure) und DHA (Docosahexaensäure) für Säuglinge und möglicherweise EPA (Eicosapentaensäure) und DHA auch für Erwachsene. Eine umfassende Aufzählung von Vitaminen findet sich in "Referenzwerte für die Nährstoffzufuhr", 1. Auflage, Umschau Braus Verlag, Frankfurt am Main, 2000, herausgegeben von der Deutschen Gesellschaft für Ernährung. Beispiele geeigneter Formulierungen zur Nahrungsergänzung sind Kapseln, Tabletten, Pillen, Pulverbeutel, Flüssigampullen und Fläschchen mit Tropfeinsätzen, sowie die oben bereits genannten Arzneiformen.

Lebensmittel-Formulierungen haben in der Regel die übliche Form und werden beispielsweise als Frühstückszubereitungen, in Form von Müslis oder Riegeln, Sportlerdrinks, Komplettmahlzeiten, diätetische Zubereitungen, wie Diätdrinks, Diätmahlzeiten und Diätriegel, angeboten.

Die Herstellung erfindungsgemäßer Nahrungsergänzungsmittel und Lebensmittel erfolgt nach dem Fachmann geläufigen Methoden und bedarf keiner weiteren Erläuterung ( vgl. z.B. Hans-Dieter Belitz et al. Lehrbuch der Lebensmittelchemie. Springer-Lehrbuch 5., überarb. Aufl. 2001. XLIV, 1059 Verlag: SPRINGER, BERLIN).

Der Gehalt an erfindungsgemäßem Wirkstoffen/Wirkstoffkombinationen in obigen Nahrungsergänzungsmitteln und Lebensmitteln kann über einen weiten Bereich variieren und liegt z. B in einem Bereich von 0,01 bis 10 Gew.-%, wie z.B. 0,1 bis 1 Gew.-%.

Die Erfindung wird nunmehr anhand der folgenden nichtlimitierenden Herstellungs- und Formulierungsbeispiele näher erläutert:

### EXPERIMENTELLER TEIL

### Allgemeine Methoden:

### Bestimmung von Stilbenen durch Hochdruckflüssigkeitschromatographie (HPLC) in Trockenextrakt aus Rhapontikrhabarberwurzel

### a) Probenvorbereitung:

50 mg Extrakt werden mit 40 ml einer Mischung aus Aceton und Wasser (1:1) in einem Braunglasgefäß versetzt, 15 Minuten im Ultraschallbad behandelt und mit der Lösungsmittelmischung auf 50 ml aufgefüllt und anschließend 1:10 mit der Lösungsmittelmischung verdünnt.

### b) Durchführung der Chromatographie:

Mit einem Teil der oben erhaltenen Lösung wird eine Hochdruckflüssigkeitschromatographie (HPLC) mit folgenden Systemparametern durchgeführt:

| | |
|---|---|
| Probenschleife: | 20µl |
| Säule: | Lichrospher 5 µ RP 18, 250 x 4 mm |
| Vorsäule: | Lichrospher 5 µ RP 18, 5 x 4 mm |
| Säulentemperatur: | 25 °C |
| Fließmittel A: | Acetonitril/dest. Wasser/Phosphorsäure 85 %, 15/85/0,05 (Volumenteile) |
| Fließmittel B: | Acetonitril/dest. Wasser/Phosphorsäure 85 % 80/20/0,05 (Volumenteile) |
| Fluss: | 1,5 ml/min |
| Säulenspülung: | 15 min mit Eluent 50 % B; Equilibrierzeit 15 min |
| Detektion: | 310 nm |
| HPLC: | Kontron Kroma 2000 |

### Gradient:

| **Zeit** | **% B** |
|---|---|
| 0,0 | 0 |
| 0,5 | 0 |
| 7,5 | 75 |
| 8,5 | 100 |
| 9,5 | 0 |
| 12,5 | 0 |

Unter den oben angegebenen Systembedingungen ergeben sich folgende Retentionszeiten:

| | |
|---|---|
| Rhaponticin: | ca. 5,5 min |
| Desoxyrhaponticin: | ca. 6,8 min |
| Rhapontigenin: | ca. 7,2 min |
| Desoxyrhapontigenin: | ca. 9,0 min |

Für eine quantitative Bestimmung werden die jeweiligen Peak-Flächen ermittelt und mit den entsprechenden Peak-Flächen eines Standard-Extraktes bekannter Zusammensetzung verglichen.

### Herstellungsbeispiel 1: Herstellung des Trockenextraktes ERr 731 aus Rhapontikrhabarberwurzel mit einer wässrigen Calciumhydroxid-Lösung

Für die Herstellung eines Trockenextraktes aus Rhapontikrhabarberwurzel werden eingesetzt:

| | |
|---|---|
| Droge (Radix rheum rhaponticum) | 50,0 kg |
| Calciumoxid | 5,0 kg |
| Gereinigtes Wasser | 190,0 kg |

Essigsäure (je nach Bedarf um den erforderlichen pH-Wert einzustellen)

Die erzielbare Ausbeute liegt dabei zwischen 2 und 3 kg je 50 kg Droge.

Die Herstellung erfolgt in folgenden Schritten:
a) In einen Plastikbottich werden zunächst 5 kg Calciumoxid gefüllt und mit 20 kg gereinigtem Wasser aufgeschlämmt. Die unter diesen Bedingungen stattfindende Bildung von Calciumhydroxid (Löschkalk) führt zu einem starken Temperaturanstieg der Lösung. Daher kann das Calciumhydroxid erst nach Abkühlen weiter verwendet werden. Die Temperatur der Lösung liegt dann bei 30°C bis 35°C.
b) 50 kg Droge werden in einen Mischer gefüllt und mit dem oben erwähnten Löschkalk versetzt. Um den Löschkalk möglichst vollständig aus dem Plastikbottich zu entfernen, wird dieser mit 10 kg gereinigtem Wasser nachgespült. Diese Waschflüssigkeit wird ebenfalls in den Mischer gegeben.
c) Die mit Löschkalk homogen durchmischte Droge wird in einen Perkolator gefüllt und mit 160 kg gereinigtem Wasser überschichtet. Der Perkolator bleibt für 48 Stunden verschlossen. Anschließend wird das Perkolat in einem geeigneten Gefäß mit einer Flussrate von 50ml/min aufgefangen. Die Perkolation wird solange fortgesetzt, bis kein Perkolat mehr austritt. Die Drogenmasse wird nach Beendigung nicht ausgepresst, sondern verworfen.
d) Unter permanenter Kontrolle wird dem Perkolat solange konzentrierte Essigsäure zugefügt, bis ein pH-Wert erreicht ist, der im Bereich von 3,4 bis 3,6 liegt. Um eine möglichst vollständige Fällung des Extraktes zu erreichen, ruht der Ansatz für 5 Tage.
e) Die Gewinnung des Trockenextraktes erfolgt durch Filtration über Büchnertrichter unter angelegtem Vakuum. Abschließend wird der Extrakt noch mit 10 bis 20 kg gereinigtem Wasser gewaschen.
f) Der nach Filtration erhaltene Trockenextrakt wird im Trockenschrank bei 40°C solange getrocknet, bis eine Restfeuchte-Toleranz von max. 1 % erreicht ist.

Rhaponticin ist in wässrigen Lösungen mit alkalischem pH-Bereich gut löslich, während es im sauren pH-Bereich (pH 3,4 - 3,6) als gelbliche Substanz ausfällt. Dies macht man sich bei seiner Isolierung zu Nutzen. Da die Wurzel neben anderen organischen Säuren vor allem einen hohen Gehalt an Oxalsäure besitzt (2/3 in wasserlöslicher und 1/3 in gebundener Form) muss diese während der Isolation neutralisiert werden, um ein Abdriften des pH-Werts in den sauren Bereich zu verhindern und so ein frühzeitiges Ausfallen des Rhaponticins zu unterbinden. Dies wird durch den Einsatz von Calciumoxid erreicht. Dieses wird als Löschkalk-Lösung mit einem pH-Wert von 12,4 - 12,6 eingesetzt.

Wird der Löschkalk mit der Droge homogen durchmischt, so verändert sich der pH-Wert der Mischung. Er liegt dann im Bereich von 11,3 bis 11,8, wodurch ein Ausfällen von Rhaponticin verhindert wird, da die phenolische Form in eine Phenolat-Form überführt wurde. Trotz des hohen Gehalts an Oxalsäure kann der pH-Wert im alkalischen Bereich gehalten werden. Dies ist darauf zurückzuführen, dass das Calciumhydroxid mit Oxalsäure reagiert und unlösliches und ungiftiges Calciumoxalat bildet.

Durch die anschließende Perkolation mit gereinigtem Wasser wird Rhaponticin aus der Wurzel extrahiert. Nach Beendigung der Perkolation wird durch Zugabe von Essigsäure ein pH-Wert von 3,4 bis 3,6 eingestellt. Dieser pH-Shift vom alkalischen in den sauren Bereich führt zum Ausfällen von Rhaponticin durch Rückführung in die phenolische Form. Um eine möglichst vollständige Fällung des Rhaponticins zu erreichen, lässt man den Ansatz 5 Tage stehen. Danach wird abfiltriert. Rhaponticin bleibt als gelbliche Substanz auf dem Filter zurück.

Obige Ausführungen zu Rhaponticin gelten entsprechend für die anderen erfindungsgemäß isolierten Hydroxystilbenwirkstoffe.

### Herstellungsbeispiel 2: Herstellung eines Trockenextraktes aus Rhapontikrhabarberwurzel mit verschiedenen organischen Lösungsmitteln

In der hier als Droge verwendeten Rhapontikrhabarberwurzel gehören die hauptsächlich nachweisbaren Inhaltstoffe zur Gruppe der Hydroxystilbene. Aus dieser Gruppe finden sich in den Wurzeln Rhaponticin (Rh) mit einem Anteil von etwa 6 % und Desoxyrhaponticin (DRh) mit einem Anteil von etwa 4 %.

Bei Einwirkung der im Folgenden angegebenen Lösungsmittelsysteme in der 100-fachen Menge, bei Raumtemperatur für 10 Minuten unter Schütteln oder Rühren lassen sich die nachfolgend zusammengefassten Anteile extrahieren:

| | | |
|---|---|---|
| **Ethanol 86 %** | Rh | 100,8 % |
| | DRh | 99,5 % |
| | | |
| **Ethanol 15 %** | Rh | 77,1 % |
| | DRh | 75,5 % |
| | | |
| **Aceton** | Rh | 88,3 % |
| | DRh | 96,6 % |
| | | |
| **Wasser, alkalisch** | Rh | 75,5 % |
| (pH 11, eingestellt mit CaO-Lösung) | DRh | 60,5 % |

Keine brauchbaren Resultate wurden mit Heptan erzielt.

Die jeweiligen Ausbeuten an Rohextrakten in Massenanteilen (bezogen auf eingesetzte Droge) sind folgende:

| | |
|---|---|
| **Ethanol 86 %** | 35,5 % |
| **Ethanol 15 %** | 32,2 % |
| **Aceton** | 21,4 % |
| **Heptan** | 0 % |
| **Wasser, alkalisch** | 4,5 % |

Die Extraktion von Rhapontikrhabarberwurzel mit Ethanol-Wasser-Gemischen führt zu einem Extrakt, der neben den Hauptinhaltsstoffen Rhaponticin (ca. 30 %) und Desoxyrhaponticin (ca. 22 %), ein weiteres z.Zt. noch nicht erforschtes Stilben in ca. 20 % Anteil am Extrakt enthält. Daneben erhält man die Aglycone Rhapontigenin (ca. 8 %) und Desoxyrhapontigenin (ca. 2 %) und noch 9 weitere Verbindungen, die sich zu ca. 20 % summieren.

Bei der Extraktion mit Aceton erhält man grundsätzlich gleiche Ergebnisse.

Die Extraktion mit alkalisiertem Wasser (vgl. Bedingungen gemäß Herstellungsbeispiel 1) führt zu einem Extrakt höherer Reinheit.

Die Hauptinhaltsstoffe Rhaponticin und Desoxyrhaponticin sind in einen ca. 97 %igen Anteil im trockenen Extrakt enthalten. Rhapontigenin und Desoxyrhapontigenin ergeben im Extrakt einen Anteil von zusammen 1,1 %, während das noch nicht erforschte Stilben lediglich in einem Anteil von 0,2 % enthalten ist. Weitere 3 Verbindungen sind in einem 2,5 %igen Anteil enthalten.

### Formulierungsbeispiel 1: Herstellung einer festen Dosierungsform -Minitablette

### 1. Herstellung des Tablettenkerns:

Ein fester Tablettenkern wird unter Verwendung folgender Wirk- und Hilfsstoffe in den angegebenen Mengenverhältnissen (TI = Gewichtsteile) hergestellt. Die Bestandteile werden auf drei verschieden Weisen vermischt und tablettiert:

### a) Rezeptur für Tablettenkern:

| Gereinigter Trockenextrakt gemäß | |
|---|---|
| Herstellungsbeispiel 1 | |
| aus Rhapontikrhabarberwurzel (ERr 731^{®}) | 3,6 TI |
| Mikrokristalline Cellulose (z.B. Avicel^{®}) | 57,0 TI (± 40 %) |
| Sorbit | 8,0 TI " |
| Talkum | 2,5 TI " |
| Makrogol 6000 (Polyglykol) | 1,6 TI " |
| Polyvidon (K-Wert ca. 25, z.B. Kollidon^{®} 25) | 1,6 TI " |
| Natriumdodecylsulfat (z.B. Texapon^{®} K 12) | 0,5 TI " |
| Magnesiumstearat (pflanzlich) | 0,8 TI " |
| | 75,6 TI (± 40 %) |

Durch Variation der eingewogenen ERr 731®-Menge und/oder Variation der mikrokristallinen Cellulose-Menge können beliebige ERr 731®-Gehalte des Rohkerns erhalten werden (wie z.B. 2, 4, 6, 8, 10, 12 mg je Tablette).

### b) Mischen von Droge und Träger

### - Mischungs-Variante a:

1,2 TI ERr 731® werden anteilig mit Avicel^{®} in der Kugelmühle verrieben, anschließend wird nach Zugabe der übrigen Hilfsstoffe wie unten beschrieben gemischt und tablettiert.

### - Mischungs-Variante b:

ERr 731® (1g/l Lösungsmittel) wird in einem geeigneten Lösungsmittel (z.B. Ethanol/Wassergemisch 86% v/v Ethanol) gelöst und auf Avicel^{®} aufgezogen, getrocknet (bei 40°C mindestens 48 Stunden) und nach Zugabe der übrigen Hilfsstoffe wie unten beschrieben gemischt und tablettiert.

### - Mischungs-Variante c:

Die gesamte Avicel®-Menge wird in drei gleiche Portionen aufgeteilt. Die erste Portion wird mit der Gesamtmenge ERr731® versetzt und in einer Laborkugelmühle (z.B. Typ 1-25 LK, Alpine, Augsburg) mindestens für 120 Minuten verrieben. Dann gibt man die zweite Portion Avicel® hinzu, verreibt erneut mindestens 120 Minuten in der Laborkugelmühle. Nach Zugabe der dritten Portion Avicel® wird nochmals kurz vermischt. Anschließend wird nach Zugabe der übrigen Hilfsstoffe wie unten beschrieben gemischt und tablettiert.

Mit dieser Mischungsvariante gelingt es überraschenderweise, die Entmischungsneigung deutlich zu verringern und selbst bei kleinen Dosiseinheiten einen äußerst gleichförmigen Wirkstoffgehalt von nicht mehr als ± 5 Gew.-% (bestimmt nach Ph. Eur. 5. Ausgabe 2005 (5.0/2.09.06.00)) einzustellen.

### c) Tablettierung:

Die Mischung aus Avicel® und Wirkstoff wird durch eine Siebmaschine (Siebdurchmesser 1.2 mm) in einen geeigneten Mischbehälter gesiebt und nach Zugabe der angegebenen Tablettierungshilfsmittel (ohne Magnesiumstearat) in einem geeigneten Mischer (z.B. Rhönrad-Mischer Typ Standard RR M 200, Firma Engelsmann AG/Ludwigshafen) mind. 30 min gemischt. Nach Zugabe von Magnesiumstearat wird nochmals mind. 5 min gemischt.

Die Verpressung erfolgt auf einer geeigneten Tablettenpresse (z.B. Rundläufer Typ Perfecta Fette 2000, Fa. Fette/Schwarzenbeck):

| | |
|---|---|
| Kerngewicht: | 84 mg ± 4.2 mg maximale Abweichung |
| Stempel: | 7 mm Durchmesser, drageegewölbt |

Der ERr-731-Gehalt je Kern beträgt etwa 4 mg ± 5%.

### 2. Herstellung der magensaftresistenten überzogenen Tablette

Nach dem Entstauben der Tablettenkerne mit Eudragit wird ein magensaftresistenter Überzug aus Celluloseacetatphthalat und Diethylphthalat, gelöst in Isopropanol und Ethylacetat, auf die Tablettenkerne mit Hilfe einer Coatinganlage aufgebracht.

Macrogol wird in gereinigtem Wasser gelöst. Die Bestandteile Zucker (Saccharose oder Isomalt), Calciumcarbonat, Talkum, Titaniumdioxid und die beiden Povidone werden gemischt und in die Flüssigkeit eingerührt. Die Suspension wird 20 Minuten im Leitstrahlmischer (z.B. Rototron Typ RTA 70-50) gerührt.

Die Dragiersuspension wird mit Hilfe eines Dragierautomaten auf die isolierten Kerne aufgebracht. Der Vorgang wird so lange wiederholt, bis ein durchschnittliches Gewicht von 150 mg pro aufdragiertem Kern erreicht ist. Schließlich trägt man das Polierwachs auf und anschließend lässt man bis zum Hochglanz rollieren.

Endgewicht der magensaftresistenten überzogenen Tablette:
150 mg ± 7.5 mg maximale Abweichung.

Auf diese Weise werden zwei verschieden Tablettenformen - eine zuckerhaltige und eine zuckerfreie - hergestellt, wobei die jeweiligen Hilfsstoffen in den im Folgenden angegebenen Gewichtsteilen eingesetzt wurden.

### a) Magensaftresistente überzogene Minitablette - zuckerhaltig - mit Weichmacher im Coating

### Hilfstoffe:

| | | |
|---|---|---|
| Coating: | Eudragit L12,5%Trockensubstanz | 1,350 kg (± 40 %) |
| | Diethylphthalat | 1,749 kg " |
| | Celluloseacetatphthalat | 7,770 kg " |
| | Isopropylalkohol | 75,600 kg " |
| | Ethylacetat | 77,600 kg " |
| | Talkum | 2,000 kg " |
| | | |
| Dragierung: | Talkum | 7,182 kg " |
| | Zucker | 28,747 kg " |
| | Calciumcarbonat | 6,410 kg " |
| | Titandioxid E 171 | 0,635 kg " |
| | Povidon | |
| | (K-Wert ca. 25, z.B. Kollidon^{®} 25) | 0,756 kg " |
| | Povidon (K-Wert: ca. 90) | 0,332 kg " |
| | Macrogol 35.000 | 0,635 kg " |
| | Wasser | 10,500 kg " |
| | | |
| Politur: | 95 % Carnaubawachs, | |
| | 5 % Gebleichtes Wachs | |
| | (z.B. Capol 1295 PH) | 0,108 kg " |

### b) Magensaftresistente überzogene Minitablette - zuckerfrei - mit Weichmacher im Coating

### Hilfstoffe:

| | | |
|---|---|---|
| Coating: | Eudragit L12,5% Trockensubstanz | 1 ,350 kg (± 40 %) |
| | Diethylphthalat | 1,749 kg " |
| | Celluloseacetatphthalat | 7,770 kg " |
| | Isopropylalkohol | 75,600 kg " |
| | Ethylacetat | 77,600 kg " |
| | | |
| Dragierung: | Talkum | 7,482 kg " |
| | Sorbit und/oder Isomalt | 28,747 kg " |
| | Calciumcarbonat | 6,410 kg " |
| | Titandioxid E 171 | 0,635 kg " |
| | Povidon | |
| | (K-Wert ca. 25, z.B. Kollidon^{®} 25) | 0,756 kg " |
| | Povidon (K-Wert: ca. 90) | 0,332 kg " |
| | Macrogol 35.000 | 0,635 kg " |
| | Wasser | 10,500 kg " |
| | | |
| Politur: | 95 % Carnaubawachs, | |
| | 5 % Gebleichtes Wachs | |
| | (z.B. Capol 1295 PH) | 0,108 kg " |

### Formulierungsbeispiel 2: Herstellung einer festen Dosierungsform - Minitablette zuckerhaltig ohne Weichmacher

### 1. Herstellung des Tablettenkerns

Die Herstellung erfolgt analog Formulierungsbeispiel 1.

### 2. Herstellung der magensaftresistenten überzogenen Tablette

Die Herstellung erfolgt analog Formulierungsbeispiel 1, wobei jedoch anstelle von Cellulsoeacetatphthalat /Diethylphthalat (Weichmacher) Schellack (Variante A) oder Aqoat (Variante B) verwendet wird.

### a) Variante A

| Hilfsstoffe: | | kg (± 40 %) |
|---|---|---|
| Coating: | Eudragit L12,5% Trockensubstanz | 0,400 |
| | CAPOL 5270 PH 8% | |
| | (Schellacklösung) | 60,000 |
| | = 4,8 kg Trockensubstanz | |
| | (Schellack) | |
| | Isopropylalkohol | 4,000 |
| | Ethanol 96% | 3,200 |
| | Talkum | ..2,000 |
| | | |
| Dragierung: | Talkum | 7,182 |
| | Zucker | 28,747 |
| | Calciumcarbonat | 6,410 |
| | Titandioxid E 171 | 0,635 |
| | Polyvidon | |
| | (K-Wert ca. 25, z.B. Kollidon^{®} 25) | 0,756 |
| | Povidon (K-Wert: ca. 90) | 0,332 |
| | Macrogol 35.000 | 0,635 |
| | Wasser | 10,500 |
| | | |
| Politur: | 95 % Carnaubawachs | |
| | 5 % Gebleichtes Wachs | |
| | (z.B. Capol 1295 PH) | 0,108 |

### b) Variante B

| Hilfsstoffe: | | kg (± 40 %) |
|---|---|---|
| Coating: | Eudragit L12,5% Trockensubstanz | 0,400 |
| | Aqoat | |
| | Hydroxypropylmethylcellulose- | |
| | acetatsuccinat | 5,420 |
| | Aqua dest. | 12,500 |
| | Isopropylalkohol | 4,000 |
| | Ethanol 86% | 55,000 |
| | | |
| Dragierung: | Talkum | 9,182 |
| | Zucker | 28,747 |
| | Calciumcarbonat | 6,410 |
| | Titanoxid E 171 | 0,635 |
| | Polyvidon | |
| | (K-Wert ca. 25, z.B. Kollidon^{®} 25) | 0,756 |
| | Povidon (K-Wert: ca. 90) | 0,332 |
| | Macrogol 35.000 | 0,635 |
| | Wasser | 10,500 |
| | | |
| Politur: | 95 % Carnaubawachs | |
| | 5 % Gebleichtes Wachs | |
| | (z.B. Capol 1295 PH) | 0,108 |

### Formulierungsbeispiel 3: Herstellung einer festen Dosierungsform - Minitablette zuckerfrei ohne Weichmacher

### 1. Herstellung des Tablettenkerns

Die Herstellung erfolgt analog Formulierungsbeispiel 1, wobei jedoch anstelle von Avicel Isomalt verwendet wurde.

### 2. Herstellung der magensaftresistenten überzogenen Tablette

Die Herstellung erfolgt analog Formulierungsbeispiel 2, wobei jedoch anstelle von Zucker Isomalt verwendet wurde.

### a) Variante A

| Hilfsstoffe: | | kg (± 40 %) |
|---|---|---|
| Coating: | Eudragit L12,5% Trockensubstanz | 0,400 |
| | CAPOL 5270 PH 8% | |
| | (Schellacklösung) | 60,000 |
| | = 4,8 kg Trockensubstanz | |
| | (Schellack) | |
| | Isopropylalkohol | 4,000 |
| | Ethanol 96% | 3,200 |
| | Talkum | 2,000 |
| | | |
| Dragierung: | Talkum | 7,182 |
| | Isomalt | 28,747 |
| | Calciumcarbonat | 6,410 |
| | Titanoxid E 171 | 0,635 |
| | Polyvidon | |
| | (K-Wert ca. 25, z.B. Kollidon^{®} 25) | 0,756 |
| | Povidon (K-Wert: ca. 90) | 0,332 |
| | Macrogol 35.000 | 0,635 |
| | Wasser | 10,500 |
| | | |
| Politur: | 95 % Carnaubawachs | |
| | 5 % Gebleichtes Wachs | |
| | (z.B. Capol 1295 PH) | 0,108 |

### b) Variante B

| Hilfsstoffe: | | kg (± 40 %) |
|---|---|---|
| Coating: | Eudragit L12,5% Trockensubstanz | 0,400 |
| | Aqoat | 5,420 |
| | Aqua dest. | 12,500 |
| | Isopropylalkohol | 4,000 |
| | Ethanol 86% | 55,000 |
| | Talkum | ..2,000 |
| | | |
| Dragierung: | Talkum | 7,182 |
| | Isomalt | 28,747 |
| | Calciumcarbonat | 6,410 |
| | Titanoxid E 171 | 0,635 |
| | Polyvidon | |
| | (K-Wert ca. 25, z.B. Kollidon^{®} 25) | 0,756 |
| | Povidon (K-Wert: ca. 90) | 0,332 |
| | Macrogol 35.000 | 0,635 |
| | Wasser | 10,500 |
| | | |
| Politur: | 95 % Carnaubawachs | |
| | 5 % Gebleichtes Wachs | |
| | (z.B. Capol 1295 PH) | 0,108 |

### Formulierungsbeispiel 4: Herstellung einer halbfesten Dosierungsform - Vaginalgel

Die Herstellung erfolgt unter Anwendung üblicher Methoden nach den beiden folgenden Varianten:

### a) Variante A:

Hydroxypropylmethylcellulose (Hypromellose USP) oder ein anderer Gelbildner wird mit 2 - 10 Gew.-% in gereinigtem Wasser quellen gelassen. Anschließend wird das in Glycerin gelöste ERr 731^{®} (Herstellungsbeispiel 1) eingearbeitet. Die Glycerinmenge kann bis zu 50 Gew.-% des Gels betragen. ERr 731^{®} kann in Glycerin bis zu 0,5 Gew.-% gelöst werden. Im Bedarfsfall können dem Gel Konservierungsstoffe (z. B. Sorbinsäure und ihre Salze) hinzugegeben werden. Auch eine pH-Einstellung ist möglich. Alternativ zu Glycerin kann auch Ethanol 30 - 86 Vol.-% verwendet werden.

### b) Variante B:

Carbomer (Carbopol) wird mit 0,5 - 5 Gew.-% in gereinigtem Wasser gelöst und der gewünschte pH-Wert (z. B. KOH, NaOH, NH₃) eingestellt. Wenn notwendig, wird ein Konservierungsmittel (z. B. Sorbinsäure und ihre Salze) beigemischt. Nach Bildung eines klaren Gels wird ERr 731^{®} (Herstellungsbeispiel 1) bis zu 0,5 Gew.-% in Ethanol 30 - 86 Vol.-% gelöst und zugegeben. Alternativ zu Ethanol kann auch Glycerin verwendet werden.

### Formulierungsbeispiel 5: Herstellung einer halbfesten Dosierungsform - Vaginalkugeln

Es werden Kugeln mit einer Größe von 1 bis 15 g mit einem Gehalt von 1 bis 12 mg ERr 731^{®} (Herstellungsbeispiel 1) gelöst in Glycerin (85 % n 20/D =1,45085) nach zwei verschiedenen Varianten in herkömmlicher Weise hergestellt.

### a) Variante A:

### Rezeptur:

| | |
|---|---|
| Gelatine | 1 Teil |
| Gereinigtes Wasser | 2 Teile |
| Glycerin 85 % (+ERr 731^{®}) | 5 Teile |

### b) Variante B:

Gleiche Rezeptur, jedoch mit geeignetem Konservierungsmittel, wie z. B. Sorbat, Benzoat, PHB-Ester.

Die Gelatine wird jeweils in gereinigtes Wasser eingebracht und quellen gelassen bis alles glasig geworden ist. Anschließend wird Glycerin 85 % mit Wirkstoff dazugegeben und erwärmt, jedoch nicht über 65 °C. Anschließend werden die Globuli in üblicher Weise gegossen.

### Formulierungsbeispiel 6: Herstellung einer flüssigen Dosierungsform - Tropfen

### a) Lösungsversuche mit ERr 731^{®} in Ethanol und Glycerol:

Gehalt des Extrakts:
61,9 % Rhaponticin
29,9 % Desoxyrphaponticin

Versuch A: 200 mg Trockenextrakt in 50 ml Glycerol R:

| | |
|---|---|
| 55,1 % Rhaponticin | (89,0 % der Theorie) |
| 27,1 % Desoxyrhaponticin | (90,6 % der Theorie) |

Versuch B: 200 mg Trockenextrakt in 50 ml Ethanol 30 % R:

| | |
|---|---|
| 52,2 % Rhaponticin | (84,3 % der Theorie) |
| 25,2 % Desoxyrhaponticin | (84,2 % der Theorie) |

Versuch C: 200 mg Trockenextrakt in 50 ml Ethanol 50 % R:

| | |
|---|---|
| 58,8 % Rhaponticin | (95,0 % der Theorie) |
| 29,0 % Desoxyrhaponticin | (97,0 % der Theorie) |

Versuch D: 200 mg Trockenextrakt in 50 ml Ethanol 86 % R:

| | |
|---|---|
| 59,8 % Rhaponticin | (96,6 % der Theorie) |
| 29,5 % Desoxyrhaponticin | (98,7 % der Theorie) |

### b) Herstellung von Tropfen:

Zur Herstellung von Tropfen wurde Trockenextrakt gemäß Versuch B in Ethanol 30% R gelöst und gegebenenfalls filtriert.

## Patentansprüche

1. Verfahren zur Herstellung eines Hydroxystilben-haltigen Drogenextraktes, wobei man
a) einen Hydroxystilben-haltigen Teil einer Drogenpflanze, gegebenenfalls in zerkleinerter Form, bereitstellt,
b) diesen mit einem wässrigen Extraktionsmittel versetzt,
c) nach Einwirken des Extraktionsmittels eine Flüssigextraktphase aus der Mischung gewinnt und die Extraktion gegebenenfalls mehrfach wiederholt, und
d) das Extraktionsmittel aus den so erhaltenen Flüssigextraktphasen entfernt,
wobei man eine Extraktion mit einem wässrigen Extraktionsmittel bei einem pH-Wert der Mischung im alkalischen Bereich durchführt.

2. Verfahren nach Anspruch 1, wobei der erhaltene Extrakt wenigstens eine Verbindung, ausgewählt unter Rhaponticin, Desoxyrhaponticin, Rhapontigenin, Desoxyrhapontigenin als Salz oder in phenolischer Form, umfasst.

3. Verfahren nach einem der vorhergehenden Ansprüche, wobei die Drogenpflanze unter Pflanzen der Gattung Rheum spp, Astragalus spp,Cassia spp oder Picea spp ausgewählt ist.

4. Verfahren nach einem der vorhergehenden Ansprüche, wobei der Hydroxystilben-haltige Teil der Drogenpflanze die Wurzel von Rheum rhaponticum ist.

5. Verfahren nach einem der vorhergehenden Ansprüche, wobei man von der Droge ein Hydroxystilben-haltiges Perkolat herstellt.

6. Verfahren nach einem der Ansprüche 1 bis 5, wobei der pH-Wert der Mischung im Bereich von etwa 11 bis 12 liegt.

7. Verfahren nach einem der Ansprüche 1 bis 6, wobei der pH-Wert der Mischung mit Hilfe einer anorganischen Base, ausgewählt unter Alkali- und Erdalkalimetallhydroxiden, eingestellt wird.

8. Verfahren nach Anspruch 7, wobei die Base Calciumhydroxid ist.

9. Verfahren nach Anspruch 8, wobei das Mengenverhältnis von vorgelegter Droge zu Calciumhydroxid (berechnet als Calciumoxid) im Bereich von etwa 5:1 bis 20:1 liegt.

10. Verfahren nach einem der Ansprüche 1 bis 9, wobei man aus der erhaltenen alkalischen Flüssigextraktphase das (die) Hydroxystilben(e) ausfällt.

11. Verfahren nach Anspruch 10, wobei man den pH-Wert des Extrakts auf einen Wert im Bereich von etwa 3 bis 4 einstellt.

12. Verfahren nach einem der Ansprüche 10 und 11, wobei man den Niederschlag abtrennt, wäscht und trocknet.

13. Verfahren nach einem der Ansprüche 1 bis 5, wobei man den Niederschlag, gegebenenfalls nach weiterer Reinigung, derivatisiert.

14. Verfahren nach Anspruch 13, wobei die Derivatisierung eine Veresterung umfasst.

15. Verfahren nach Anspruch 13, wobei die Derivatisierung eine Veretherung umfasst.

16. Verfahren zur Herstellung eines eines Mittels ausgewählt unter Arzneimitteln, anderen Arzneipflanzenzubereitungen, Nahrungsergänzungsmitteln und/oder diätetischen Lebensmitteln, wobei man
a) einen Hydroxystilben-haltigen Drogenextrakt unter Anwendung eines Verfahrens nach einem der vorhergehenden Ansprüche herstellt; und
b) diesen Extrakt zu dem Mittel formuliert,

17. Verfahren nach Anspruch 16, wobei das Arzneimittel eine feste, halbfeste oder flüssige Dosierungsform umfasst.

18. Verfahren nach Anspruch 17, wobei die feste Dosierungsform einen wirkstoffhaltigen festen Kern mit einem pharmazeutisch verträglichen Träger umfast und einen Wirkstoffgehalt von etwa 1 bis 20 Gew.-%, bezogen auf das Gesamtgewicht des Kerns, aufweist, wobei der Wirkstoff wenigstens eine Verbindung, ausgewählt unter Rhaponticin, Desoxyrhaponticin, Rhapontigenin, Desoxyrhapontigenin, sowie deren stereoisomeren Formen, jeweils als Salz oder in phenolischer Form, sowie Kombinationen von wenigstens zwei dieser Verbindungen, und zusätzlich gegebenenfalls wenigstens eine Verbindung, ausgewählt unter Resveratrol, Piceatannol, Astringin, deren stereoisomere Formen, jeweils als Salz oder in phenolischer Form, umfasst.

19. Verfahren nach Anspruch 18, wobei die Wirkstoffkombination im wesentlichen Rhaponticin und Desoxyrhaponticin in einem Gewichtsverhältnis von etwa 2:1 bis 1:2 umfasst.

20. Verfahren nach einem der Ansprüche 18 und 19, wobei die Wirkstoffkombination im wesentlichen aus etwa
60-66 Gew.-% Rhaponticin
30-35 Gew.-% Desoxyrhaponticin
0-2 Gew.-% Rhapontigenin und
0-2 Gew.-% Desoxyrhapontigenin
besteht.

21. Verfahren nach einem der Ansprüche 17 bis 20, wobei die Dosierungsform einem Gesamt-Wirkstoffgehalt von etwa 2 bis 20 mg je Dosiseinheit aufweist.

22. Verfahren nach einem der Ansprüche 17 bis 21, wobei die feste Dosierungsform einen Lactose-freien Kern aufweist.

23. Verfahren nach einem der Ansprüche 17 bis 22, wobei die feste Dosierungsform in Form einer Pille, einer Tablette, eines Extrudats oder eines Granulats vorliegt.

24. Verfahren nach einem der Ansprüche 17 bis 23, wobei die feste Dosierungsform einen magensaftresistenten Überzug aufweist.

25. Verfahren nach Anspruch 17, wobei das Arzneimittel ausgewählt ist unter Gelen und Lösungen.

26. Verfahren zur Herstellung eines pharmazeutischen Mittels zur Behandlung von klimakterischen Beschwerden der Frau, juveniler Oligomenorrhoe und Dysmenorrhoe, primärer und sekundärer Amenorrhoe oder Endometritis, von Prostatakrebs und Erkrankungen des unteren Urogenitaltrakts, von Krebs, von chronischen entzündlichen Erkrankungen, von Depressionen und Angst, von Osteoporose sowie von Kopfschmerzen und Migräne, wobei man
a) einen Hydroxystilben-haltigen Drogenextrakt unter Anwendung eines Verfahrens nach einem der Ansprüche 1 bis 15 herstellt; und
b) diesen Extrakt zu dem Mittel formuliert.

27. Verfahren nach Anspruch 26, zur Herstellung eines pharmazeutischen Mittels zur Behandlung von klimakterischen Beschwerden in der Peri- oder Postmenopause.

28. Verfahren nach einem der Ansprüche 26 und 27, zur Herstellung eines pharmazeutischen Mittels zur Behandlung von Hitzewallungen, Schweißausbrüchen, Schlafstörungen, Reizbarkeit, physischer und mentaler Erschöpfung, Sexualproblemen und Harntraktbeschwerden.

29. Verfahren nach Anspruch 28, zur Herstellung eines pharmazeutischen Mittels zur Behandlung von klimakterischen Beschwerden aufgrund natürlicher oder therapeutisch verursachter Menopause.

## Claims

1. Process for preparing a drug extract containing hydroxystilbene, wherein
a) a hydroxystilbene-containing part of a medicinal plant is provided, optionally in comminuted form,
b) this is combined with an aqueous extraction agent,
c) after the extraction agent has acted, a liquid extract phase is recovered from the mixture and the extraction is optionally repeated several times, and
d) the extraction agent is eliminated from the liquid extract phases thus obtained,
wherein extraction is carried out with an aqueous extraction agent, the pH of the mixture being in the alkaline range.

2. Process according to claim 1, wherein the extract obtained comprises at least one compound selected from among rhaponticin, desoxyrhaponticin, rhapontigenin, desoxyrhapontigenin as a salt or in phenolic form.

3. Process according to one of the preceding claims, wherein the medicinal plant is selected from among plants of the genus Rheum spp, Astragalus spp, Cassia spp or Picea spp.

4. Process according to one of the preceding claims, wherein the hydroxystilbene-containing part of the medicinal plant is the root of Rheum rhaponticum.

5. Process according to one of the preceding claims, wherein a hydroxystilbene-containing percolate is prepared from the drug.

6. Process according to one of claims 1 to 5, wherein the pH of the mixture is in the range from about 11 to 12.

7. Process according to one of claims 1 to 6, wherein the pH of the mixture is adjusted using an inorganic base selected from among alkali metal and alkaline earth metal hydroxides.

8. Process according to claim 7, wherein the base is calcium hydroxide.

9. Process according to claim 8, wherein the ratio of drug introduced to calcium hydroxide (calculated as calcium oxide) is in the range from about 5:1 to 20:1.

10. Process according to one of claims 1 to 9, wherein the hydroxystilbene(s) is (are) precipitated from the alkaline liquid extract phase obtained.

11. Process according to claim 10, wherein the pH of the extract is adjusted to a value in the range from about 3 to 4.

12. Process according to one of claims 10 and 11, wherein the precipitate is separated, washed and dried.

13. Process according to one of claims 1 to 5, wherein the precipitate is derivatised, optionally after further purification.

14. Process according to claim 13, wherein the derivatisation comprises an esterification.

15. Process according to claim 13, wherein the derivatisation comprises an etherification.

16. Process for preparing an agent selected from among medicaments, other medicinal plant preparations, food supplements and/or dietetic foods, wherein
a) a hydroxystilbene-containing drug extract is prepared using a process according to one of the preceding claims; and
b) this extract is formulated to produce the agent.

17. Process according to claim 16, wherein the medicament comprises a solid, semisolid or liquid dosage form.

18. Process according to claim 17, wherein the solid dosage form comprises a solid core containing an active substance with a pharmaceutically acceptable carrier and an active substance content of about 1 to 20 % by weight, based on the total weight of the core, wherein the active substance comprises at least one compound selected from among rhaponticin, desoxyrhaponticin, rhapontigenin, desoxyrhapontigenin, and the stereoisomeric forms thereof, in each case as a salt or in phenolic form, and combinations of at least two of these compounds, and additionally optionally at least one compound selected from among resveratrol, piceatannol, astringin, the stereoisomeric forms thereof, in each case as a salt or in phenolic form.

19. Process according to claim 18, wherein the active substance combination essentially comprises rhaponticin and desoxyrhaponticin in a ratio by weight of about 2:1 to 1:2.

20. Process according to one of claims 18 and 19, wherein the active substance combination consists essentially of about
60-66 % by weight of rhaponticin,
30-35 % by weight of desoxyrhaponticin,
0-2 % by weight of rhapontigenin and
0-2 % by weight of desoxyrhapontigenin.

21. Process according to one of claims 17 to 20, wherein the dosage form has a total active substance content of about 2 to 20 mg per dosage unit.

22. Process according to one of claims 17 to 21, wherein the solid dosage form has a lactose-free core.

23. Process according to one of claims 17 to 22, wherein the solid dosage form is present as a pill, a tablet, an extrudate or a granulate.

24. Process according to one of claims 17 to 23, wherein the solid dosage form has a coating resistant to gastric acids.

25. Process according to claim 17, wherein the medicament is selected from among gels and solutions.

26. Process for preparing a pharmaceutical agent for treating climacteric complaints in women, juvenile oligomenorrhoea and dysmenorrhoea, primary and secondary amenorrhoea or endometritis, prostate cancer and diseases of the lower urogenital tract, cancer, chronic inflammatory diseases, depression and anxiety, osteoporosis and headaches and migraine, wherein
a) a hydroxystilbene-containing drug extract is prepared using a process according to one of claims 1 to 15; and
b) this extract is formulated to produce the agent.

27. Process according to claim 26, for preparing a pharmaceutical agent for treating climacteric complaints in the peri- or post-menopause.

28. Process according to one of claims 26 and 27, for preparing a pharmaceutical agent for treating hot flushes, sweats, sleep disorders, irritability, physical and mental exhaustion, sexual problems and urinary tract complaints.

29. Process according to claim 28, for preparing a pharmaceutical agent for treating climacteric complaints due to naturally occurring or therapeutically induced menopause.

## Revendications

1. Procédé de préparation d'un extrait de drogue contenant de l'hydroxystilbène, dans lequel on
a) met à disposition une partie contenant de l'hydroxystilbène d'une plante à drogue, sous forme fragmentée le cas échéant,
b) on la met en contact avec un agent extracteur aqueux,
c) après l'action de l'agent extracteur, on récupère du mélange une phase liquide d'extraction et on répète l'extraction le cas échéant plusieurs fois, et
d) on élimine l'agent extracteur des phases d'extrait liquide ainsi obtenu
sachant que l'on exécute l'extraction avec un agent extracteur aqueux à un pH du mélange dans la plage alcaline.

2. Procédé selon la revendication 1, dans lequel l'extrait obtenu comprend au moins un composé, choisi parmi la rhaponticine, la désoxyrhaponticine, la rhapontigénine, la désoxyrhapontigénine, comme sel ou sous forme phénolique.

3. Procédé selon l'une quelconque des revendications précédentes, dans lequel la plante à drogue est choisie parmi les plantes des espèces Rheum spp, Astragalus spp, Cassia spp ou Picea spp.

4. Procédé selon l'une quelconque des revendications précédentes, dans lequel la partie de la plante à drogue contenant l'hydroxystilbène est la racine de Rheum rhaponticum.

5. Procédé selon l'une quelconque des revendications précédentes, dans lequel on prépare à partir de la drogue un extrait contenant de l'hydroxystilbène obtenu par percolation.

6. Procédé selon l'une quelconque des revendications 1 à 5, dans lequel le pH du mélange est compris dans l'intervalle d'environ 11 à 12.

7. Procédé selon l'une quelconque des revendications 1 à 6, dans lequel le pH du mélange est ajusté à l'aide d'une base inorganique, choisie parmi les hydroxydes alcalins et alcalinoterreux.

8. Procédé selon la revendication 7, dans lequel la base est de l'hydroxyde de calcium.

9. Procédé selon la revendication 8, dans lequel le rapport quantitatif de la drogue mise en jeu à l'hydroxyde de calcium (calculé comme oxyde de calcium) est compris dans l'intervalle d'environ 5 : 1 à 20 : 1.

10. Procédé selon l'une quelconque des revendications 1 à 9, dans lequel on précipite le(s) hydroxystilbène(s) de la phase alcaline liquide d'extrait obtenue.

11. Procédé selon la revendication 10, dans lequel on ajuste le pH de l'extrait à une valeur dans l'intervalle d'environ 3 à 4.

12. Procédé selon l'une quelconque des revendications 10 et 11, dans lequel on sépare le précipité, on le lave et on le sèche.

13. Procédé selon l'une quelconque des revendications 1 à 5, dans lequel on produit des dérivés du précipité, le cas échéant après une purification supplémentaire.

14. Procédé selon la revendication 13, dans lequel la production de dérivé comprend une estérification.

15. Procédé selon la revendication 13, dans lequel la production de dérivé comprend une éthérification.

16. Procédé de préparation d'un moyen choisi parmi les produits pharmaceutiques, d'autres produits de plantes médicinales, des compléments alimentaires et/ou des denrées alimentaires diététiques, dans lequel on
a) prépare un extrait de drogue contenant de l'hydroxystilbène moyennant l'application d'un procédé d'après l'une quelconque des revendications précédentes ; et
b) on formule cet extrait pour donner le moyen.

17. Procédé selon la revendication 16, dans lequel le produit pharmaceutique comprend une forme de dosage solide, semi-solide ou liquide.

18. Procédé selon la revendication 17, dans lequel la forme de dosage solide comprend un noyau solide contenant le principe actif avec un support pharmaceutiquement acceptable et présente une teneur en principe actif d'environ 1 à 20 % en poids, rapportée au poids total du noyau, le principe actif comportant au moins un composé choisi parmi la rhaponticine, la désoxyrhaponticine, la rhapontigénine, la désoxyrhapontigénine, ainsi que leurs formes stéréo-isomères, respectivement comme sel ou sous forme phénolique, ainsi que des combinaisons d'au moins deux de ces composés, et en plus le cas échéant au moins un composé, choisi parmi le resvératrol, le picéatannol, l'astringine, leurs formes stéréo-isomères, respectivement comme sel ou sous forme phénolique.

19. Procédé selon la revendication 18, dans lequel la combinaison de principes actifs comporte essentiellement de la rhaponticine et de la désoxyrhaponticine dans un rapport pondéral d'environ 2 : 1 à 1 : 2.

20. Procédé selon l'une quelconque des revendications 18 et 19, dans lequel la combinaison de principes actifs consiste essentiellement en environ
60 à 66 % en poids de rhaponticine,
30 à 35 % en poids de désoxyrhaponticine,
0 à 2 % en poids de rhapontigénine et
0 à 2 % en poids de désoxyrhapontigénine.

21. Procédé selon l'une quelconque des revendications 17 à 20, dans lequel la forme de dosage comporte une teneur totale en principes actifs d'environ 2 à 20 mg par unité de dosage.

22. Procédé selon l'une quelconque des revendications 17 à 21, dans lequel la forme de dosage solide comporte un noyau exempt de lactose.

23. Procédé selon l'une quelconque des revendications 17 à 22, dans lequel la forme de dosage solide se présente sous la forme d'une pilule, d'un comprimé, d'un solide extrudé ou d'un granulé.

24. Procédé selon l'une quelconque des revendications 17 à 23, dans lequel la forme de dosage solide comporte un revêtement résistant au suc gastrique.

25. Procédé selon la revendication 17, dans lequel le produit pharmaceutique est choisi parmi des gels et des solutions.

26. Procédé pour la préparation d'un moyen pharmaceutique pour le traitement des incommodités climatériques de la femme, de l'oligoménorrhée et de la dysménorrhée juvéniles, de l'aménorrhée primaire et secondaire ou de l'endométrite, du cancer de la prostate et des affections du tractus urogénital inférieur, du cancer, des affections inflammatoires chroniques, des dépressions et des angoisses, de l'ostéoporose ainsi que des maux de tête et des migraines, dans lequel on
a) prépare un extrait de drogue contenant de l'hydroxystilbène moyennant l'application d'un procédé selon l'une quelconque des revendications 1 à 15 et
b) on formule cet extrait pour obtenir ledit moyen.

27. Procédé selon la revendication 26, pour la préparation d'un moyen pharmaceutique pour le traitement des incommodités climatériques dans la péri- et dans la post-ménopause.

28. Procédé selon l'une quelconque des revendications 26 et 27 pour la préparation d'un moyen pharmaceutique pour le traitement des bouffées de chaleur, des hyperhidroses, des troubles du sommeil, de l'irritabilité, de l'épuisement physique et mental, des problèmes sexuels et des incommodités du tractus urinaire.

29. Procédé selon la revendication 28 pour la préparation d'un moyen pharmaceutique pour le traitement d'incommodités climatériques par suite de la ménopause naturelle ou provoquée thérapeutiquement.
